Europäisches Patentamt

European Patent Office

Office européen des brevets

(11) Veröffentlichungsnummer : **0 113 038**
**B1**

(12) **EUROPÄISCHE PATENTSCHRIFT**

(45) Veröffentlichungstag der Patentschrift :
10.09.86

(21) Anmeldenummer : 83111779.1

(22) Anmeldetag : 24.11.83

(51) Int. Cl.⁴ : **C 07 C 93/193, C 08 F 20/34**

(54) **Verfahren zur Herstellung konzentrierter wässriger Lösungen von Quaternisierungsprodukten von tertiären Aminoalkylestern oder tertiären Aminoalkylamiden der Acryl- oder Methacrylsäure.**

(30) Priorität : 30.11.82 DE 3244274

(43) Veröffentlichungstag der Anmeldung :
11.07.84 Patentblatt 84/28

(45) Bekanntmachung des Hinweises auf die Patenterteilung : 10.09.86 Patentblatt 86/37

(84) Benannte Vertragsstaaten :
BE DE FR GB NL

(56) Entgegenhaltungen :
US-A- 3 948 979

(73) Patentinhaber : BASF Aktiengesellschaft
Carl-Bosch-Strasse 38
D-6700 Ludwigshafen (DE)

(72) Erfinder : Schneider, Kurt, Dr.
Auf dem Köppel 28
D-6702 Bad Duerkheim (DE)
Erfinder : Heide, Wilfried, Dr.
Laerchenweg 4
D-6701 Erpolzheim (DE)
Erfinder : Hartmann, Juergen, Dr.
Bruesseler Ring 47
D-6700 Ludwigshafen (DE)
Erfinder : Hartmann, Heinrich, Dr.
Weinheimer Strasse 46
D-6703 Limburgerhof (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der DE-B-28 48 627 ist ein Verfahren zur Herstellung eines flüssigen, unmittelbar polymerisierbaren Gemisches aus Acrylamid und Quaternierungsprodukten von tertiären Aminoalkylestern oder tertären Aminoalkylamiden der Acryl- oder Methacrylsäure bekannt. Auf das Gemisch, das 30 bis 80 Gew.% Acrylamid enthält, läßt man das Alkylierungsmittel einwirken. Man erhält eine Schmelze, deren Schmelzpunkt nicht unterhalb von 50 °C liegt. Solche Schmelzen sind technisch schwierig zu handhaben, weil Transportleitungen und Pumpen auf eine Temperatur geheizt werden müssen, die oberhalb des Schmelzpunktes der Gemische liegt.

Aufgabe der Erfindung ist es, ein Verfahren zur Herstellung konzentrierter wäßriger Lösungen von Quaternierungsprodukten von tertiären Aminoalkylestern oder tertiären Aminoalkylamiden den Acryl- oder Methacrylsäure durch Reaktion der entsprechenden Ester oder Amide mit einem Alkylierungsmittel in einem wasserlöslichen Keton als Lösungsmittel zur Verfügung zu stellen, bei dem leicht handhabbare wäßrige Lösungen der Quaternierungsprodukte anfallen, die keine anderen Monomeren enthalten und die direkt bei der Polymerisation eingesetzt werden können.

Die Aufgabe wird erfindungsgemäß dadurch gelöst, daß man zur Isolierung der Quaternierungsprodukte soviel Wasser zum Reaktionsgemisch zusetzt, daß sich zwei Phasen ausbilden und die untere wäßrige Phase, die die Quaternierungsprodukte gelöst enthält, von der oberen, Keton und restliches Alkylierungsmittel enthaltenden Phase abtrennt. Dem Reaktionsgemisch wird dabei soviel Wasser zugesetzt, daß 50 bis 95 Gew.%ige wäßrige Lösungen der Quaternierungsprodukte entstehen. Die so erhaltenen konzentrierten wäßrigen Lösungen der basischen Monomeren liegen in so großer Reinheit vor, daß sie ohne zusätzlichen Reinigungsschritt direkt bei Polymerisationen zur Herstellung von Homopolymerisaten oder auch Copolymerisaten eingesetzt werden können.

Bei der Quaternierungsreaktion können allgemein tertiäre Aminoalkylester oder tertiäre Aminoalkylamide der Acrylsäure oder Methacrylsäure eingesetzt werden. Besondere Bedeutung für die Herstellung von Homo- und Copolymerisaten haben beispielsweise Di-$C_1$-$C_2$-Alkylamino-$C_2$-$C_6$-Alkylacrylate oder die entsprechenden Methacrylate sowie Di-$C_1$-$C_2$-Alkylamino-$C_2$-$C_6$-Alkylacrylamide oder die entsprechenden Methacrylamide. Geeignete Ausgangsprodukte für die Quaternierungsreaktion sind beispielsweise Dimethylaminoethylacrylat, Dimethylaminoethylmethacrylat, Diethylaminoethylacrylat, Diethylaminoethylkmethacrylat, Dimethylaminopropylacrylat, Dimethylaminopropylmethacrylat, Diethylaminopropylacrylat, Diethylaminopropylmethacrylat, Dimethylaminoisopropylacrylat, Dimethylaminoisopropylmethacrylat, Dimethylamino-n- oder -iso-butylacrylat, Dimethylamino-n-butylmethacrylat, Dimethylamino-n-pentylacrylat, Dimethylamino-n-pentylmethacrylat, Dimethylaminoneopentylacrylat, Dimethylaminoneopentylmethacrylat, Dimethylaminohexylacrylat und Dimethylaminohexylmethacrylat sowie die entsprechenden basischen Acrylamide und Methacrylamide wie Ethylacrylamid, Diethylaminoethylmethacrylamid, Dimethylaminopropylacrylamid, Dimethylaminopropylmethacrylamid, Dimethylaminoisopropylacrylamid, Dimethylaminoisopropylmethacrylamid, Diethylaminoisopropylacrylamid, Diethylaminoisopropylmethacrylamid, Dimethylamino-n- oder -iso-butylacrylamid, Dimethylamino-n- oder -iso-butylmethacrylamid, Diethylamino-n-butylacrylamid, Dimethylamino-neopentylacrylamid, Dimethylamino-neopentylmethacrylamid, Dimethylaminohexylacrylamid und Dimethylaminohexylmethacrylamid.

Als Alkylierungsmittel kommen Alkylhalogenide in Betracht, wobei sich bezüglich der Auswahl der Alkylierungsmittel nur dadurch eine Einschränkung ergibt, daß die entstehenden Quaternierungsprodukte in Wasser löslich sein müssen. Geeignete Alkylierungsmittel sind beispielsweise Alkylhalogenide, wie Methylchlorid, Methylbromid, Methyliodid, Ethylchlorid, Ethylbrodmid, Ethyliodid, Butylchlorid, Butylbromid und Benzylchlorid. Vorzugsweise verwendet man für die Quaternierung der basischen Ester oder Amide Methylchlorid.

Da die Quaternierungsprodukte von tertiären Aminoalkylestern oder tertiären Aminoalkylamiden der Acrylsäure oder Methacrylsäure im Gegensatz zu den nicht quaternierten Produkten feste kristalline Substanzen sind, ist es für die Durchführung der Umsetzung erforderlich, ein bei Raumtemperatur flüssiges Verdünnungsmittel zu verwenden, damit während der Reaktion eine gute Durchmischung der Komponenten gewährleistet ist. Als Lösungsmittel werden bei Raumtemperatur flüssige Ketone verwendet, z. B. Aceton, Methylethylketon, Diethylketon, Methylisopropylketon und Diisopropylketon. Von diesen Ketonen verwendet man vorzugsweise Aceton oder Methylethylketon. Die Durchführung der Quaternierungsreaktion der in Betracht kommenden basischen Ester oder Amide in Aceton ist Stand der Technik (DE-B-28 48 627). Die Quaternierung kann in einem weiten Temperaturbereich durchgeführt werden, z. B. bei Raumtemperatur bis zu Temperaturen von 200 °C. Sofern man Temperaturen anwendet, die oberhalb des Siedepunktes des Lösungsmittels liegen, wird die Reaktion in einem Autoklaven durchgeführt. Ebenso empfiehlt es sich, bei Verwendung von Methylchlorid als Quaternierungsmittel bei höheren Temperaturen unter Druck zu arbeiten. Die Umsetzung mit Methylchlorid in Aceton wird vorzugsweise bei Temperaturen in dem Bereich von 20 bis 150, vorzugsweise 40 bis 100 °C durchgeführt. Es ist selbstverständlich auch möglich, durch Einleiten von Methylchlorid in eine Lösung des basischen Esters oder Amids der Acrylsäure oder Methacrylsäure in einem Keton und Raumtemperatur die Quaternierung vorzunehmen. Die Konzentration der basischen Ester oder Amide der Acryl- oder Methacrylsäure in einem Keton oder auch in einer Mischung von Ketonen beträgt 5 bis 80, vorzugsweise 20 bis 60 Gew.%. Bei der Quaternierung fallen — egal unter welchen Bedingungen sie durchgeführt wurde — feste Produkte an, die in Aceton oder den anderen wasserlöslichen ketonischen Lösungsmitteln unlöslich sind. Die Konzentration der Ausgangsstoffe in den Ketonen wird so gewählt, daß Reaktionsgemische entstehen, die noch gerührt werden können.

Zur Isolierung der Quaternierungsprodukte wird dem Reaktionsgemisch Wasser zugesetzt. Überraschenderweise bilden sich dabei zwei Phasen aus. Dieser Sachverhalt war nicht vorherzusehen, weil anzunehmen war, daß die mit Wasser mischbaren Ketone und die wasserlöslichen Quaternierungsprodukte eine homogene Lösung bilden. Dem Reaktionsgemisch wird eine derartige Menge an Wasser zugesetzt, daß sich 50 bis 95, vorzugsweise 75 bis 85 Gew.%ige wäßrige Lösungen der Quaternierungsprodukte bilden. Die untere wäßrige Phase enthält die Quaternierungsprodukte in geloster Form sowie geringe Mengen an wasserlöslichem Keton. Die so entstehenden wäßrigen Lösungen der Quaternierungsprodukte liegen in einer so hohen Reinheit vor, daß sie ohne einen zusätzlichen Reinigungsschritt direkt bei Polymerisationsreaktionen eingesetzt werden können. Die obere Phase, die nach Zugabe von Wasser zum Reaktionsgemisch entsteht, enthält das Keton und gegebenenfalls das restliche Alkylierungsmittel. Das Molverhältnis kann bei der Alkylierung in einem weiten Bereich schwanken. Man verwendet beispielsweise auf ein Mol der zu quaternisierenden Verbindung 1 bis 3 Mol, vorzugsweise 1 bis 1,5 Mol an Alkylierungsmittel. Ein nicht zu unterschätzender Vorteil des erfindungsgemäßen Verfahrens besteht darin, daß man die obere Phase, die im wesentlichen aus Keton und nicht umgesetzten Alkylierungsmitteln besteht, ohne irgendeinen Aufarbeitungsschritt durchführen zu müssen, direkt wieder bei der Quaternierungsreaktion einsetzen kann. Die Ketone, die als Lösungsmittel verwendet werden, können wasserfrei sein oder, falls sie schon einmal bei dem erfindungsgemäßen Verfahren eingesetzt wurden, bis zu 5 Gew.% Wasser enthalten. Der Wassergehalt ist selbstverständlich abhängig von dem jeweils verwendeten Keton. Vorzugsweise ist das Keton wasserfrei oder enthält nicht mehr als 1 Gew.% Wasser. Um eine vorzeitige Polymerisation der basischen Monomeren während der Quaternisierungsreaktion zu vermeiden, empfiehlt es sich, die Quaternisierung bei möglich tiefen Temperaturen und gegebenenfalls noch zusätzlich in Anwesenheit von Polymerisationsinhibitoren durchzuführen.

Es bedarf keiner näheren Ausführungen, daß bei tieferen Temperaturen längere Reaktionszeiten und bei höheren Temperaturen kurze Reaktionszeiten in Betracht kommen, und daß das Verfahren sowohl kontinuierlich als auch diskontinuierlich durchgeführt werden kann.

Die konzentrierten wäßrigen Lösungen, die erfindungsgemäß erhalten werden, werden direkt als Monomer zur Herstellung von Polymerisaten verwendet. Man kann daraus sowohl Homopolymerisate als auch Copolymerisate herstellen. Besongere Bedeutung haben Copolymerisate aus Acrylamid und Dimethylaminoethylacrylat, Copolymerisate aux Acrylamid und Dimethylaminoethylmethacrylat, Copolymerisate aus Acrylamid und Dimethylaminoethylacrylamid, Copolymerisate aus Acrylamid und Dimethylaminoethylmethacrylamid sowie die entsprechenden Copolymerisate von Diethylaminoethylacrylat mit Acrylamid und/oder Methacrylamid, wobei die basischen Monomeren erfindungsgemäß hergestellt werden und in quaternisierter Form vorliegen, vorzugsweise als Methochloride. Die Copolymerisate können außerdem Acrylsäure oder Methacrylsäure einpolymerisiert enthalten. Sie werden vorzugsweise als Flockungsmittel bei der Schlammentwässerung und Abwasserklärung verwendet.

Die in den Beispielen angegebenen Teile sind Gewichtsteile, die Angaben in Prozent sind Gew.%.

## Beispiel 1

In einem 10 l fassenden Autoklaven, der gerührt werden kann, werden 5,53 kg Aceton, 1,29 kg Dimethylaminoethylacrylat und 1,3 g Hydrochinonmonomethylether als Stabilisator vorgelegt. Dann verschließt man den Autoklav und erhitzt den Autoklaveninhalt unter Rühren auf eine Temperatur von 50 °C. Bei dieser Temperatur werden kontinuierlich 0,97 kg Methylchlorid in einem Maße zugepreßt, daß der Druck im Autoklaven etwa 3 bar beträgt. Nach Zugabe des Methylchlorids läßt man das Reaktionsgemisch noch 3 Stunden bei 50 °C nachreagieren und kühlt es dann auf Raumtemperatur ab. Dann fügt man 0,75 kg Wasser zum Reaktionsgemisch und rührt es noch 1/2 Stunde. Nach dem Abstellen des Rührers trennt es sich in 5,50 kg einer oberen, organischen Phase und 2,50 kg einer unteren, wäßrigen Phase, die farblos ist, ca. 15 Gew.% Aceton und das mit Methylchlorid quaternierte Dimethylaminoethylacrylat gelöst enthält, das direkt bei Polymerisationsreaktionen als Monomer eingesetzt werden kann.

## Beispiel 2

In einem mit einem Rührer ausgestatteten Kessel werden 1 094 g wasserfreies Aceton, 200 g Dimethylaminoethylacrylat und 0,2 g Hydrochinonmonomethylether vorgelegt und unter Rühren auf 55 °C erhitzt. Man gibt dann innerhalb von 2 Stunden 152 g Ethylbromid zu, rührt das Reaktionsgemisch nach Beendigung der Zugabe des Ethylbromids noch 12 Stunden bei 55 °C und kühlt es dann auf 25 °C ab. Dann setzt man 784 g Wasser zum Reaktionsgemisch hinzu, rührt es 15 Minuten und stellt dann den Rührer ab. Man erhält 416 g einer wäßrigen Phase, die das quaternierte Dimethylaminoethylacrylat gelöst enthält. Die wäßrige Lösung kann direkt bei der Polymerisation als Monomerphase eingesetzt werden.

# 0 113 038

## Beispiel 3

In einem mit einem Rührer ausgestatteten Kessel werden 1 165 g Aceton, das 0,2 Gew.% Wasser enthält, 200 g Dimethylaminoethylacrylat und 0,2 g Hydrochinonmonomethylether vorgelegt und auf 60 °C erhitzt. Das Reaktionsgemisch wird gerührt, wobei man innerhalb von 1 Stunde 177 g Benzylchlorid zutropfen läßt. Die Umsetzung ist nach 15 Stunden bei 60 °C beendet. Man läßt das Reaktionsgemisch abkühlen, gibt Wasser hinzu, bis sich 2 Phasen ausbilden und trennt dann 487 g einer wäßrigen Lösung ab, die das benzylierte Dimethylaminoethylacrylathydrochlorid enthält und die bei der Polymerisation direkt eingesetzt werden kann.

Herstellung von Polymerisaten

### A. Wasser-in-Öl-Polymeremulsionen

Allgemeine Herstellvorschrift :

In einem mit Rührer, Thermometer und einem Stickstoffein- und Stickstoffauslaß versehenen Behälter mischt man die Komponenten der Ölphase, deren Zusammensetzung in Tabelle 1 angegeben ist. In diese Mischung wird dann die wäßrige Monomerphase eingerührt, deren Zusammensetzung ebenfalls in Tabelle 1 angegeben ist. Man leitet 30 Minuten lang Stickstoff durch die Emulsion und erhitzt sie dann innerhalb von 15 Minuten auf eine Temperatur von 50 °C. Bei dieser Temperatur setzt man eine Lösung des Starters in wenig Aceton zu (Art und Menge vgl. Tabelle 1). Die Temperatur der Emulsion wird 2 Stunden in dem Bereich von 50 bis 55 °C gehalten. Danach wird noch einmal die gleiche Menge des Polymerisationsinitiators zugesetzt und 2 Stunden bei 55 °C nachpolymerisiert. Man erhält eine coagulatfreie und sedimentationsstabile Wasser-in-Öl-Polymeremulsion.

(Siehe Tabelle Seite 5 f.)

4

Tabelle 1

Zusammensetzung der Wasser-in-Öl-Emulsionen

| | | Bsp. 4 | Vgl.Bsp. 1 | Bsp. 5 | Vgl.Bsp. 2 |
|---|---|---|---|---|---|
| **Ölphase:** | | | | | |
| Kohlenwasserstoffgemisch (Siedebereich 192 - 254°C)[1] | [g] | 260 | 260 | 235 | 235 |
| Emulgator (Umsetzungsprodukt aus 1 Mol Oleylglycidyl-ether, 1 Mol Glycerin und 2 Mol Ethylenoxid) | [g] | 25 | 25 | 25 | 25 |
| Reaktionsprodukt aus 1 Mol Nonylphenol und 10 Molen Ethylenoxid | [g] | 5 | 5 | - | - |
| **Monomerphase:** | | | | | |
| Destilliertes Wasser | [g] | 140 | 185 | 190 | 325 |
| 70%ige wäßrige Lösung des Methochlorids von Dimethylaminoethylacrylat, erhalten gem. Bsp. 1 | [g] | 150 | - | 450 | - |
| Methochlorid von Dimethylaminoethylacrylat, Pulver, frei von Aceton | [g] | - | 105 | - | 315 |
| 50%ige wäßrige Acrylamidlösung | [g] | 390 | 390 | 70 | 70 |
| 40%ige wäßrige Lösung des Pentanatriumsalzes der Diethylentriaminpentaessigsäure | [g] | 0,23 | 0,23 | 0,04 | 0,04 |
| 37,5%ige Schwefelsäure | [g] | 0,38 | 0,38 | 0,38 | 0,38 |
| Ameisensäure | [g] | 0,30 | 0,30 | - | - |
| 2,2'-Azobis(2,4-dimethylvaleronitril), 30%ig in Aceton | [ml] | 2x0,25 | 2x0,25 | 2x0,29 | 2x0,29 |

1) Mischung aus 84 % gesättigten aliphatischen und 16 % naphtenischen Kohlenwasserstoffen

B. Perlförmige Polymerisate, hergestellt durch umgekehrte Suspensionspolymerisation

Allgemeine Vorschrift :

In einem 2 l fassenden Rundkolben, der mit Tropftrichter, Rührer, Rückflußkühler und Anschluß an eine Vakuumpumpe versehen ist, werden 800 ml Cyclohexan und 1,1 g eines Copolymeren aus Dicyclopentadien, Maleinsäureanhydrid und Styrol vorgelegt. Der Kolbeninhalt wird auf 50 °C erhitzt und der Druck so weit erniedrigt, daß die Mischung bei 50 °C siedet. Zu der Vorlage wird dann eine Mischung aus der in den folgenden Beispielen und Vergleichsbeispielen beschriebenen Monomerlösung und der dort jeweils genannten Initiatorlösung innerhalb von einer Stunde zugetropft. Die Mischung wird unmittelbar vor dem Zulauf durch Spülen mit Stickstoff von restlichem Sauerstoff befreit. Nach beendeter Zugabe der Monomeren wird das Reaktionsgemisch eine Stunde bei etwa 50 °C nachpolymerisiert, wobei die Siedekühlung aufrecht erhalten wird. Danach stellt man den Druckausgleich her und gibt 5 ml einer 2,5 %igen wäßrigen Lösung von Hydroxylammoniumsulfat, gemischt mit 0,95 ml einer 10 %igen Natronlauge zur Suspension zu. Auf den Rundkolben wird dann ein Rückflußkühler mit Wasserabscheider gesetzt und das in den Polymerteilchen befindliche Wasser durch azeotropes Auskreisen bei Normaldruck entfernt. Man erhält perlförmige Polymerisate mit einem Durchmesser von ca. 0,5 mm, die abfiltriert und im Vakuumtrockschrank bei 40 °C getrocknet werden.

Beispiel 6

Zusammensetzung der Monomerlösung :

120 g Wasser, 61,5 g einer 70 %igen wäßrigen Lösung von Dimethylaminoethylacrylat, das mit Methylchlorid quaterniert ist und gemäß Beispiel 1 erhalten wurde, 63 g Acrylamid, 0,3 g Pentanatriumsalz der Diethylentriaminpentaessigsäuren und 60 mg Ameisensäure. Als Initiatorlösung verwendete man 8,8 ml einer 3 %igen Lösung von 2,2-Azobis-(2-aminopropan)-dihydrochlorid in Wasser.

Beispiel 7

Zusammensetzung der Monomerlösung :

52 g Wasser, 198 g einer 70 %igen wäßrigen Lösung von Dimethylaminoethylacrylat, das mit Methylchlorid quaterniert ist und gemäß Beispiel 1 hergestellt wurde, 15 g Acrylamid, 0,1 mg Pentanatriumsalz der Diethylentriaminpentaessigsäure und 40 mg Ameisensäure.
Als Initiatorlösung setzte man 3,3 ml einer 3 %igen wäßrigen Lösung von 2,2-Azobis-(2-amidinopro-pan)-dihydrochlorid ein.

Vergleichsbeispiel 3

Zusammensetzung der Monomerlösung :

138 g Wasser, 43 g kristallisiertes Dimethylaminoethylacrylat, das mit Methylchlorid quaterniert ist und kein Aceton enthält, 63 g Acrylamid, 0,3 mg Pentanatriumsalz der Diethylentriaminpentaessigsäure und 60 mg Ameisensäure.
Als Initiatorlösung wurde die in Beispiel 6 beschriebene Initiatorlösung verwendet.

Vergleichsbeispiel 4

Zusammensetzung der Monomerlösung :

111 g Wasser, 139 g kristallisiertes Dimethylaminoethylacrylat, das mit Methylchlorid quaterniert und frei von Aceton ist, 15 g Acrylamid, 0,1 g Pentanatriumsalz der Diethylentriaminpentaessigsäure und 40 mg Ameisensäure.
Die im Beispiel 7 angegebene Initiatorlösung wurde als Starter verwendet.
Anwendungstechnische Prüfung der Polymerisate.
Um die Wirksamkeit der Polymerisate gemäß den Beispielen 4 und 5 und den Vergleichsbeispielen 1 und 2 zu prüfen, wurden aus den Wasser-in-Öl-Polymeremulsionen wäßrige Polymerlösungen hergestellt, indem man die Emulsionen jeweils in Wasser einrührte, das, bezogen auf die Polymeremulsion, 2 % eines mit 10 Mol Ethylenoxid umgesetzten Nonylphenols enthielt. Anschließend wurde soviel Wasser zugefügt, daß der Polymergehalt der wäßrigen Lösung 0,1 % betrug. Mit diesen Lösungen wurden die anwendungstechnischen Prüfungen an einem Faulschlamm aus einer kommunalen Kläranlage durchgeführt. Hierbei wurde die Flockungsmittelmenge bestimmt, die zu dem Schlamm gegeben werden mußte, um eine optimale Flockung zu erzielen. Die Flockung wurde visuell beurteilt.
Für die Polymerisate gemäß Beispiel 4 und Vergleichsbeispiel 1 benötigte man für eine optimale Flockung jeweils 200 mg Polymerisat/l Schlamm. Die gleiche Menge wurde von den Polymerisaten benötigt, die gemäß Beispiel 5 und Vergleichsbeispiel 2 erhalten wurde. Das bedeutet, daß die gemäß Beispiel 1 erhaltenen basischen Monomeren, die von der Herstellung her noch Verunreinigungen, insbesondere Aceton enthalten, Polymerisate mit gleicher Wirksamkeit ergeben, wie die entsprechenden reinen und acetonfreien basischen Monomeren.

An dem gleichen Faulschlamm wurde die Flockungswirksamkeit der Polymerisate getestet, die gemäß den Beispielen 6 und 7 und den Vergleichsbeispielen 3 und 4 hergestellt wurden. Für eine optimale Flockung, die visuell beurteilt wurde, benötigte man in allen Fällen 200 mg Polymerisat/l Schlamm.

Tabelle 2

| Polymerisat gemäß | Zusammensetzung | K-Wert[1] | Flockungs- test |
|---|---|---|---|
| Bsp. 6 | 60 % Acrylamid/40 % DEA-EA-CH₃Cl [2] | 219 | 20C g/m³ |
| Vgl.Bsp. 3 | 60 % Acrylamid/40 % DEA-EA-CH₃Cl | 217 | 20C g/m³ |
| Bsp. 7 | 10 % Acrylamid/90 % DEA-EA-CH₃Cl | 210 | 200 g/m³ |
| Vgl.Bsp. 4 | 10 % Acrylamid/90 % DEA-EA-CH₃Cl | 212 | 200 g/m³ |

1) bestimmt nach H. Fikentscher, Cellulose Chemie *13*, 58 bis 64 und 71 bis 74 (1932) 0,1 %ig in 5 %iger wäßriger Kochsalzlösung bei einer Temperatur von 20 °C gemessen ; dabei bedeutet $K = k \cdot 10^3$.

2) DEA—EA—CH₃Cl = Dimethylaminoethylacrylat, mit Methylchlorid quaterniert.

**Patentansprüche**

1. Verfahren zur Herstellung konzentrierter wäßriger Lösungen von Quaternierungsprodukten von tertiären Aminoalkylestern oder tertiären Aminoalkylamiden der Acryl- oder Methacrylsäure durch Reaktion der entsprechenden Ester oder Amide mit einem Alkylierungsmittel in einem wasserlöslichen Keton als Lösungsmittel, dadurch gekennzeichnet, daß man zur Isolierung der Quaternierungsprodukte soviel Wasser zum Reaktionsgemisch zusetzt, daß sich zwei Phasen ausbilden und die untere wäßrige Phase, die die Quaternierungsprodukte gelöst enthält, von der oberen, Keton und restliches Alkylierungsmittel enthaltenden Phase abtrennt.

2. Verfahren nach Anspruch 1, dadurch gekennzeichnet, daß man dem Reaktionsgemisch soviel Wasser zusetzt, daß 50 bis 95 Gew.%ige wäßrige Lösungen der Quaternierungsprodukte entstehen.

3. Verfahren nach den Ansprüchen 1 und 2, dadurch gekennzeichnet, daß man Di-C₁-C₂-Alkylamino-C₂-C₆-Alkylacrylate oder die entsprechenden -Methacrylate mit Alkylchloriden in Aceton quaterniert.

4. Verfahren nach den Ansprüchen 1 und 2, dadurch gekennzeichnet, daß man Di-C₁-C₂-Alkylamino-C₂-C₆-Alkylacrylamide oder die entsprechenden -Methacrylamide mit Alkylchloriden quaterniert.

5. Verwendung der konzentrierten wäßrigen Lösungen, die nach dem Verfahren der Ansprüche 1 bis 4 erhalten werden, als Monomer zur Herstellung von Polymerisaten.

**Claims**

1. A process for the preparation of a concentrated aqueous solution of a quaternization product of a tertiary aminoalkyl ester or tertiary aminoalkylamide of acrylic or methacrylic acid by reaction of the corresponding ester or amide with an alkylating agent in a water-soluble ketone as solvent, wherein, in order to isolate the quaternization product, water is addet to the reaction mixture in such an amount that two phases form, and the lower aqueous phase, which contains the quaternization product in solution, is separated from the upper phase, which contains the ketone and residual alkylating agent.

2. A process as claimed in claim 1, wherein water is added to the reaction mixture in such an amount that a 50-95 % strength by weight aqueous solution of the quaternization product is formed.

3. A process as claimed in claims 1 and 2, wherein a di-C₁-C₂-alkylamino-C₂-C₆-alkyl acrylate or the corresponding methacrylate is quaternized with an alkyl chloride in acetone.

4. A process as claimed in claims 1 and 2, wherein a di-C₁-C₂-alkylamino-C₂-C₆-alkylacrylamide or the corresponding methacrylamide is quaternized with an alkyl chloride.

5. The use of a concentrated aqueous solution obtained by a process as claimed in claims 1 to 4 as a monomer for the preparation of polymers.

**Revendications**

1. Procédé de préparation de solutions aqueuses concentrées de produits de quaternisation d'esters

7

aminoalkyliques tertiaires ou d'aminoalkylamides tertiaires de l'acide acrylique ou méthacrylique, par réaction des esters ou amides correspondants avec un agent d'alkylation dans une cétone hydrosoluble servant de solvant, caractérisé en ce que pour l'isolement des produits de quaternisation, on ajoute au mélange réactionnel autant d'eau qu'il en faut pour qu'il se forme deux phases et que la phase inférieure aqueuse, qui contient en solution les produits de quaternisation, se sépare de la phase supérieure, contenant la cétone et l'agent d'alkylation résiduel.

2. Procédé selon la revendication 1, caractérisé en ce qu'on ajoute au mélange réactionnel autant d'eau qu'il en faut pour qu'il en résulte des solutions aqueuses à 50-95 % en poids des produits de quaternisation.

3. Procédé selon la revendication 1 ou 2, caractérisé en ce qu'on quaternise, par des chlorures d'alkyle dans l'acétone, des acrylates de di-$C_1$-$C_2$-alkylamino-$C_2$-$C_6$-alkyle ou les méthacrylates correspondants.

4. Procédé selon la revendication 1 ou 2, caractérisé en ce qu'on quaternise, par des chlorures d'alkyle, des di-$C_1$-$C_2$-alkylamino-$C_2$-$C_6$-alkylacrylamides ou les méthacrylamides correspondants.

5. Utilisation des solutions aqueuses concentrées qui sont obtenues par le procédé de l'une quelconque des revendications 1 à 4, comme monomère pour la préparation de polymères.